# EUROPEAN PATENT SPECIFICATION

(11) **EP 2 831 284 B1**
(45) Date of publication and mention of the grant of the patent: **03.07.2019**
(21) Application number: 13770217.1
(22) Date of filing: 15.03.2013
(51) Int. Cl.: C12Q 1/6806

(54) **COMPOSITIONS AND METHODS FOR THE COLLECTION AND ISOLATION OF NUCLEIC ACIDS FROM BIOLOGICAL SPECIMENS SUSPECTED OF CONTAINING MYCOBACTERIUM TUBERCULOSIS.**
ZUSAMMENSETZUNGEN UND VERFAHREN ZUR SAMMLUNG UND ISOLIERUNG VON NUCLEINSÄUREN AUS BIOLOGISCHEN PROBEN DIE IN VERDACHT STEHEN MYCOBACTERIUM TUBERCULOSIS ZU ENTHALTEN.
COMPOSITIONS ET PROCÉDÉS POUR LA COLLECTE ET L'ISOLEMENT D'ACIDES NUCLÉIQUES À PARTIR DE SPÉCIMENS BIOLOGIQUES SUSPECTÉS DE CONTENIR MYCOBACTERIUM TUBERCULOSIS.

(30) Priority: 28.03.2012 US 201261616676 P
(43) Date of publication of application: 04.02.2015
(73) Proprietor: Longhorn Vaccines & Diagnostics, LLC, Bethesda, MD 20814 (US)
(72) Inventor: FISCHER, Gerald, W., Bethesda, MD 20817 (US); DAUM, Luke, T., San Antonio, TX 78209 (US)
(74) Representative: HGF Limited
(86) International application number: PCT/US2013/032354
(87) International publication number: WO 2013/148346

(56) References cited:
- WO-A1-03/095646
- WO-A2-01/14590
- WO-A2-2009/085355
- US-A1- 2003 143 566
- US-A1- 2007 043 216
- US-A1- 2009 312 285
- US-A1- 2011 281 754
- US-A1- 2012 252 008
- US-A1- 2013 065 223
- ROHLAND N ET AL: "Comparison and Optimization of Ancient DNA Extraction and Supplementary Material", BIOTECHNIQUES, INFORMA HEALTHCARE, US, vol. 42, no. 3, 1 March 2007 (2007-03-01), pages 343-352,11, XP007915476, ISSN: 0736-6205, DOI: 10.2144/000112383
- BOOM R ET AL: "RAPID AND SIMPLE METHOD FOR PURIFICATION OF NUCLEIC ACIDS", JOURNAL OF CLINICAL MICROBIOLOGY, AMERICAN SOCIETY FOR MICROBIOLOGY, US, vol. 28, no. 3, 1 March 1990 (1990-03-01), pages 495-503, XP000607196, ISSN: 0095-1137

## Description

### Field of the Invention

The invention is directed to an aqueous composition for use in extracting *Mycobacterium tuberculosis* nucleic acids from biological samples and to a method of detecting the presence or absence of a nucleotide sequence, gene, or genome in a biological sample suspected of containing *Mycobacterium tuberculosis.*

### Background of the Invention

The ability to maintain the integrity of nucleic acids in a biological sample (and in particular, those contained in diagnostic samples obtained from human patients), whether the specimen is taken in a remote field location, a doctor's office or in a laboratory, often determines whether the nucleic acids can be successfully analyzed. Typically, nucleic acids in a biological sample will quickly degrade and/or denature at ambient temperatures. Although some degree of degradation still occurs over time, specimens are generally stored under freezing temperatures to preserve their stability and sequence fidelity for later identification. This problem is magnified when a specimen is collected at a remote field site, or a significant distance from a doctor's office or laboratory environment, and especially where there may be limited or no access to refrigerator/freezer conditions. Problems associated with the collection and handling of biological specimens are further exacerbated when the desired nucleic acids for downstream analysis include ribonucleic acid (RNA), which is particularly susceptible to degradation by endogenous or exogenous nuclease activity.

Commercially-available specimen transport systems exist, but require refrigeration and/or special transport media for transport of biological samples from the point of collection to the point of analysis. This media imposes impossible limitations on the time a sample may be stored, or requires continual sample maintenance at low temperatures making certain collections unfeasible. Also, there are often additional concerns regarding the handling and/or storage of reagents used in storing and transporting the collected samples. For example, the reagents themselves frequently require cold temperatures or other special care to maintain stability. Due to these stability issues, for example, transport of the reagents to a field site, storage at the field site before use, and transport of the biological specimens and reagents back to a testing site is a primary concern.

Another concern when working with biological specimens is the risk of release of infectious agents to individuals and the environment, and also, of contamination to the biological specimen itself. Individuals involved in the collection, transfer, and testing process including innocent bystanders are at risk of exposure to highly dangerous contagions. As a result, the required safety measures typically increase the expense and effort required to move such samples from one location to another.

Clinical laboratory methods for pathogen detection were labor-intensive, expensive processes that required highly knowledgeable and expert scientists with specific experience. The majority of clinical diagnostic laboratories employed the use of traditional culturing methods that typically require many days for a viral culture--and even longer for some other bacterial targets. Furthermore, traditional culturing requires collection, transport, and laboratory propagation and handling of potentially infectious biological agents such as Ebola, avian influenza, severe acute respiratory syndrome (SARS), and many others.

Molecular diagnostics has changed drastically with the advent of polymerase chain reaction (PCR) and thereafter with real-time PCR. Nucleic-acid based detection platforms employing *e.g*., quantitative real-time PCR (qPCR) or reverse transcriptase PCR (RT-PCR) and quantitative, real-time, reverse transcriptase PCR (qRT-PCR) assays can deliver results in hours *versus* days required for traditional culture and isolation methods making molecular detection methods the mainstay of modern diagnostic laboratory analysis. Recent improvements in detection chemistries, such as new and improved reporting/quenching fluors, minor groove binders (MGB) (TaqMan MGB™, Applied Biosystems; for a general reference, see also *e.g.,* Baraldi et al., Pure Appl. Chem., 75(2-3):187-194, 2003), and stabilized amplification reagents have paved the way for more sensitive and highly-specific nucleic acid detection assays, and have proved more timely, robust, and economical than antiquated cell culture-based methods. Advances in other nucleic acid detection strategies such as transcription-mediated amplification, ligase chain reaction (LCR), and so-called "laboratory-on-a-chip" multiplexed assays, have also contributed to the transition from culture vials to microarrays in the clinical laboratory.

Several commercial companies (*e.g*., Qiagen [Valencia, CA, USA], Roche Applied Science [Indianapolis, IN, USA], Gen-Probe [San Diego, CA, USA], and bioMérieux [Durham, NC, USA]) have developed instruments to automate the nucleic acid extraction process from sample isolation to molecular analysis. For example, the Tigris DTS® (Gen-Probe, San Diego, CA, USA) automates the entire detection process, and in late 2004 was approved by the U.S. Food and Drug Administration (FDA) for simultaneously detecting *Chlamydia trachomatis* and *Neisseria gonorrhoeae* using Gen-Probe's APTIMA COMBO-2® amplified nucleic acid test (NAT) assay WO2009/085355 discloses the PrimeStore™ Solution. US2011/281754 discloses alternative compositions and methods to detect *Mycobcaterium* nucleic acid.

In view of the requirement for high-quality nucleic acid samples in contemporary detection and assay systems, there is a need in the art for safe and facile collection, storage and transport systems that do not require refrigeration or freezing or special handling, yet maintain the integrity and quality of nucleic acids contained within a variety of biological samples and specimens. Moreover, this need is high-lighted when the specimen is a harmful or pathogenic organism. Furthermore, there is now a need for less-expensive, and more-convenient collection/transport/storage media: (i) that minimizes the risk of pathogen exposure to workers and others; (ii) that provides concentration; (iii) that facilitates convenient transportation of biological specimens for extended periods of time at ambient temperatures; and (iv) such that the sample can be appropriately analyzed as necessary for a variety of macromolecules including nucleic acids, genes and genomes.

### Summary of the Invention

The present invention encompasses an aqueous composition for use in extracting and detecting Mycobacterium tuberculosis nucleic acids from biological samples.

The invention is directed to a composition comprising. 4 M guanidine thiocyanate; about 30 mM sodium citrate; about 0.25% (wt./vol.) sodium dodecyl sulfate and about 0.25% (wt./vol.) *N*-lauroyl sarcosine, sodium salt; about 0.1 M 2-mercaptoethanol; a defoaming agent of about 0.1% silicone polymer (wt./vol.); and NACM materials such as, for example, preferably silica-coated porcelain, ceramic, plastic, or glass beads.

Another embodiment of the invention is directed to a method for obtaining a population of nucleic acids from a sample suspected of containing *Mycobacterium tuberculosis* comprising in one step contacting the sample with an amount of the a composition of the invention under conditions effective to detect a desired population of nucleic acids from the sample that is specific to an identifiable gene, genome, pathogen of other desired sequence. Preferably, the sample comprises one or more viral, bacterial, fungal, animal, or plant cells that are physically lysed upon contact with the composition. Also preferably, the integrity of the population of nucleic acids in the sample is at least substantially maintained when the composition comprising the sample is stored at a temperature of from about 15C to about 40°C for a period of from about 7 to about 14 days. Preferably, the composition comprising the sample is stored substantially at ambient temperature from the time of collection to the time of analyzing the population of nucleic acids therein.

### Description of the Invention

Extended stabilization, collection, transport, preservation and isolation compositions are in great demand as the ability to screen large populations for diseases and disorders is increasing. Samples or specimens are often located in geographical regions that are remote from a testing facility. Remote locations, also referred to as field sites, encompass a variety of environments where diagnostic testing is typically not performed. These sites include doctors' offices, triage centers, airports, border crossings, outbreak areas, and a variety of outdoor locations.

It has been surprisingly discovered that desired macromolecules and preferably nucleic acids can be obtained from individuals and small and large populations by collecting the biological sample is a biological specimen collection composition containing affinity chromatography matrix materials. These compositions extract, preserve and maintain the high-quality and high-fidelity (e.g., sequence fidelity of nucleotides, deoxynucleotides, sequences, genes or genomes) of populations of the macromolecules of interest from the biological material. The macromolecules are spared the deleterious effects routinely observed in conventional samples that are subjected to degradation and contamination during the multiple steps involved with the collection, isolation, storage, and transport processes. Macromolecules that can be detected by the compositions and methods of the invention include nucleic acids, DNA, and RNA. Nucleic acid sequences that can be detected and/or identified include, but are not limited to genes, genomes, cancer marker sequences, sequences indicating the presence of *Mycobacterium tuberculosis* or infection, sequences indicating a phenotypic condition of *Mycobacterium tuberculosis,* sequencing indicating a lineage, sequences indicating identifiable characteristics, sequences indicating a mutation, sequences indicating a change from a wild-type or other know sequence, or a combination thereof.

One embodiment of the invention is directed to an extraction, collection and preservation composition for the collection of biological specimens suspected of containing *Mycobacterium tuberculosis* such that living material contained therein is killed or otherwise rendered harmless by the composition itself so as to pose no or minimal risk of exposure, while at the same time the macromolecules are released for analysis from membranes and other structures that otherwise require additional steps for removal and separation. The composition also destroys or renders sufficiently inactive enzymes that may be present in the sample that often degrade nucleic acids. The composition also facilitates the release of DNA/RNA from cells, cell membranes, sub-cellular structures and/or other structures present in the sample. Preferably, the released nucleic acids bind to a detectable affinity matrix material, also a preferred component of the composition, for ease of isolation and/or analysis of the nucleic acids. Preferably, the composition and the sample are maintained in a single reaction vessel, such that the integrity of the nucleic acids to be detected is at least sufficiently maintained for later diagnostic analysis. Thus, in one-step, the composition of the invention simultaneously: inactivates or kills pathogens that may be present in the sample; inhibits or prevents enzymatic degradation of nucleic acids of the sample that are targeted for later identification, facilitates the release of target nucleic acids from cells and cell structures present in the sample , facilitates separation of released nucleic acids and maintains the integrity of the nucleic acids for later analysis. Preferably, the composition containing the sample does not require refrigeration or freezing and, more preferably, can be maintained at ambient temperatures throughout the sample collection process and at all times prior to analysis. Optionally, the composition may contain predetermined nucleic acid sequences that can serve as one or more internal positive controls (IPC) or one or more internal negative controls (INC) to monitor fidelity, accuracy and/or obtain qualitative and/or quantitative information from the subsequent analysis. Also optionally, compositions may contain carrier nucleic acid such as DNA or RNA.

The ability to achieve all of these desirable functions in a single-step formulation, preferably in a single reaction zone or reaction vessel, is a particularly marked advantage over that presently available. Presently, existing technologies do not include a single-step composition that provides for inactivation of biological components from *Mycobacterium tuberculosis* containing nucleic acids, release of the nucleic acids through chemical and/or physical lysis of cells followed by separation or release of RNA/DNA, maintenance of the integrity of the liberated population of nucleic acids, facilitate the isolation of the desired nucleic acids for later analysis, and IPCs and/or INCs to further quantify nucleic acids in the sample. The present invention both stabilizes and preserves the integrity of the nucleic acids from the sample for diagnostic testing, while also providing convenient tools for isolating, concentrating and monitoring, and analyzing targets within the sample. Thus, the compositions of the invention are ideal for clinical, field and deployment use, or for high volume sample collection and extraction procedures. Biological samples collected in compositions of the invention are biologically inactivated, and therefore, may be safely shipped and stored, typically without refrigeration or freezing.

Macromolecules of the sample that can be detected include nucleic acids such as DNA and RNA or any identifiable sequences therein. Preferably the nucleic acids to be detected in the collected samples are nucleic acid from a conserved sequence that is indicative of an infection or disorder, or for screening purposes.

Compositions of the invention contain one or more chaotropes; one or more detergents; one or more reducing agents; one or more chelators; one or more buffers; a defoaming agent, and one or more nucleic acid capture matrix (NACM) materials, together present in an amount sufficient to denature proteins, inactivate enzymes, kill pathogens, and not degrade nucleic acids of a sample suspected of containing selected nucleic acid sequences when a sample is contacted with the composition; wherein: a) the one or more chaotropes comprise 4 M guanidine thiocyanate; b) the one or more detergents comprise 0.25% (wt./vol.) sodium dodecyl sulfate and 0.25% (wt./vol.) N-lauroyl sarcosine, sodium salt; c) the one or more reducing agents comprise 0.1 M 2-mercaptoethanol; d) the one or more chelators comprise 30 mM sodium citrate; e) the one or more buffers are present in an amount from 0.0001% to 0.3% (wt./vol.) or from 1 mM to 1M; 1) the defoaming agent comprises 0.1% silicone polymer (wt./vol.); and (g) the one or more NACM materials which comprise polymer, porcelain, ceramic, plastic, polymer or glass beads coated with a nucleic acid binding substance, or a combination thereof, and are present in an amount from 1 ng to 10 µg per 100 µL.

Exemplary nucleic acid capture matrix (NACM) materials include, preferably, agarose, glass, cellulose, polyacrylamide, sepharose, sephadex, silica, or another matrix media. Preferably, the NACM material is coated with a nucleic acid binding substance (NABS), such as, for example, nucleic acid (NA) binding proteins, antibodies and chemicals with an affinity for NAs including single-stranded nucleic acid sequences. NACM include materials that are coupled to specific antibodies or antibody fragments or other macromolecules or ligands that facilitate extract and/or isolation of the diagnostic molecule of interest. Affinity beads are preferably magnetic beads such as, for example, beads commercially available from Dynabeads®, Life Technologies; TurboBeads, TurboBeads Inc. or PureProteome™, and Millipore. Beads may contain pores of defined sizes that are useful for inclusion or exclusion molecular size chromatography. NACM materials includes matrix media such as, for example, resins that are useful with the compositions and method of the invention such as, preferably, amino acid resins, carbohydrate resins, ion exchange resins, and hydrophobic and hydrophilic resins. The presence of matrix material in the composition of the invention serve to facilitate the release and subsequent capture of macromolecules from the cells, cell structures, macromolecules, and biological and non-biological debris of the sample. Preferably, these same matrix materials can then serve to expedite the isolation of the macromolecules for later analysis through magnetic attraction, molecular affinity, ionic or non-ionic interactions, density or specific density, hydrophobic or hydrophilic interactions, shape, color or light emission or absorption or any unique or identifiable distinguishing chemical or physical property.

Optionally, the compositions of the invention may further include one or more buffers (each preferably present in the final composition in an amount from about 1 mM to about 1 M). Exemplary buffers include, preferably, tris(hydroxymethyl) aminomethane (Tris), citrate, 2-(N-morpholino)ethanesulfonic acid (MES), *N,N*-bis(2-hydroxyethyl)-2-aminoethanesulfonic acid (BES), 1,3-bis(tris(hydroxymethyl)methylamino)propane (Bis-Tris), 3-(cyclohexylamino)-1-propanesuhinic acid (CAPS), 3-(cyclohexylamino)-2-hydroxy-1-propanesuhicic acid (CAPSO), 4-(2-hydroxyethyl)-1-piperazineethanesulfonic acid (HEPES), 3-(*N*-morpholino)propanesulfonic acid (MOPS), 3-(*N*-morpholino)-2-hydroxypropanesulfonic acid (MOPSO), piperazine-N,N\'-bis(2-hydroxypropanesulfonic acid (POPSO), *N*-[Tris(hydroxymethyl)methyl]-3-amino propanesulfonic acid (TAPS), *N*-[Tris(hydroxymethyl)methyl]-3-amino-2-hyidroxypropansulfonic acid (TAPSO), *N*-[Tris(hydroxymethyl)methyl]-2-aminoethanesulfonic acid (TES), *N,N*-bis(2-hydroxyethyl) glycine (Bicine), *N*-[tris(hydroxymethyl)methyl]glycine (Tricine), *N*-2-acetamido-2-iminodiacetic acid (ADA), *N*-(2-acetamido)-2-aminoethanesulfonic acid (ACES), piperazine-1,4-bis(2-ethanesulfonic acid) (PIPES), bicarbonate, phosphate, or any combination thereof. In a preferred embodiment, the buffer includes a citrate.

The inclusion of one or more of such optional but preferred buffers is desirable to control the pH of the formulations, since it has been found that nucleic acid extraction is optimal in a pH range of about 5 to 8. Preferably, the one or more buffers employed in the disclosed compositions are chosen to provide a significant buffering capacity in the range from a pH of about 5.5 to about 7.5, more preferably within a pH range of about 6 to about 7, and more preferably still, within a pH range of about 6.2 to about 6.8. In exemplary embodiments, the pH of a PrimeStore™ solution (also referred to herein as "PSS") is preferably about 6.9 ± 0.25.

The compositions of the invention may also further optionally include one or more short-chain (preferably from 1- to 6-carbon [*i.e.,* C₁-C₆] alcohols) alkanols (each preferably present in the composition in an amount from about 1% to about 25%, although higher percentages of the alcohols may be employed if desired). Exemplary short-chain alkanols include linear and branched-chain alcohols, such as, without limitation, methanol, ethanol, propanol, butanol, pentanol, hexanol, or any combination thereof.

The compositions of the invention may also further optionally include one or more additional compounds or reagents including, without limitation, cationic functionalized zwitterionic compounds such as betaines (including, without limitation, *N,N,N-*trimethylglycine, cocamidopropyl betaine, and the like), albuminoids (including, without limitation, ovalbumin, and the serum albumins of bovine, equine, or human origin), and osmolytes (including, without limitation, trimethylamine *N*-oxide (TMAO), dimethylsulfoniopropionate, sarcosine, and saccharides or sugar alcohols including, without limitation, trehalose, maltose, rhamnose, sucrose, arabinose, fucose, mannitol, sorbitol, adonitol, and the like).

Preferably, the compositions of the invention provide sufficient buffering capacity to adequately stabilize the populations of polynucleotides obtained from a sample, and will, most preferably, be buffered to a pH of about 6.4 to 6.9 during formulation, and will maintain the isolated populations of polynucleotides in a similar pH range when the sample is contacted with the storage/collection formulations described herein. Buffering capacity of the composition is preferably maximized by matching the buffer's pKa value with the pH of the composition. Variations between pKa and pH are preferably equal to or less than ±1.0, ±0.75, ±0.5 or ±0.25.

The compositions of the present invention will typically at least substantially inactivate, and preferably entirely inactivate, any endogenous or exogenous RNAses, DNAses or proteases present in the sample, such that the macromolecules of the sample are substantially free of any degradation, and preferably do not degrade, or lose integrity, during the collection, lyses, concentration, storage, and transport of the sample for subsequent *in vitro* analyses.

Other formulations of the storage/transport/collection compositions not part of the invention are described in the examples herein, and include, without limitation, a composition that includes about 4 M of a chaotrope (such as guanidine thiocyanate, guanidine hydrochloride, guanidine isocyanate, or any combination thereof), about 10 mM to 30 mM of a chelator (such as EGTA, HEDTA, DTPA, NTA, EDTA, citrate anhydrous, sodium citrate, calcium citrate, ammonium citrate, ammonium bicitrate, citric acid, diammonium citrate, ferric ammonium citrate, lithium citrate, or any combination thereof), about 0.25% of a detergent (such as SDS, LDS, NaTDC, NaTC, NaGC, NaDC, sodium cholate, NaABS, NLS, or any salt or combination thereof), about 0.1 M of a reducing agent (such as β-ME, DTT, DMSO, formamide, TCEP, or any combination thereof), about 0.1% of a surfactant/defoaming agent (such as a silicone polymer [e.g., Antifoam A®] or a polysorbate [*e.g.,* Tween®], or any combination thereof), and about 1.0 µg/100µl of magnetic affinity beads.

Additional formulations of the specimen collection compositions herein disclosed include, without limitation, a composition that includes about 3 M of a chaotrope (such as guanidine thiocyanate, guanidine hydrochloride, guanidine isocyanate, or any combination thereof), about 1 mM of 0.5 M reducing agent (such as, *e.g.,* β-ME, TCEP, formamide, DTT, DMSO, or any combination thereof), about 1 to about 10 mM of a chelator (such as, *e.g.,* EGTA, HEDTA, DTPA, NTA, EDTA, citrate anhydrous, sodium citrate, calcium citrate, ammonium citrate, ammonium bicitrate, citric acid, diammonium citrate, ferric ammonium citrate, lithium citrate, or any combination thereof), about 0.25% of a detergent (such as SDS, LDS, NaTDC, NaTC, NaGC, NaDC, sodium cholate, NaABS, NLS, or any salt or combination thereof), and optionally but preferably about 0.0002% of a defoaming agent (also referred to as an antifoaming agent) (such as a silicone polymer or a polysorbate, or any combination thereof), about 100 mM of a buffer (such as Tris, MES, BES, Bis-Tris, HEPES, MOPS, bicarbonate, citrate, phosphate, or any combination thereof), and about 1.0 µg/100µl of magnetic affinity beads.

Another formulation of the disclosed polynucleotide isolation and stabilization compositions include, without limitation, a composition that includes about 1 to about 4 M of a chaotropic agent such as guanidine thiocyanate, guanidine hydrochloride, or guanidine isocyanate; about 0.5 to 100 mM of a chelating agent such as EDTA, or sodium citrate, or both; about 0.1 to about 1% of an anionic detergent such as SDS or *N*-lauroyl sarcosine, sodium salt; about 0.001 to about 0.0001% of a surfactant or wetting agent such as the silicone polymer, Antifoam A®, e); about 10 to about 500 mM of a buffering agent such as Tris-HCl; about 10 to about 25% of a short-chain alkanol such as ethanol, and about 1.0 µg/100µl of magnetic affinity beads.

Here is also described a composition that includes about 2.5 M guanidine thiocyanate; about 0.5 mM TCEP; about 10 mM sodium citrate; about 0.4% *N*-lauroyl sarcosine, sodium salt; about 0.0002% Antifoam A, about 100 mM Tris-HCl, about 0.1 mM EDTA; about 23% ethanol, and about 1.0 µg/100µl of magnetic affinity beads.

The invention also provides a method for obtaining a population of nucleic acids from a sample containing *Mycobacterium tuberculosis.* The method generally involves associating the sample with an amount of one of the compositions of the invention under conditions effective to obtain a population of nucleic acids from the sample. The invention optionally includes a facilitated release of the DNA/RNA from cells or cell structures of the biological sample and an affinity concentration of the desired population of nucleic acids for later nucleic acid and/or sequence analysis.

The present disclosure also describes a method of preparing a one-step aqueous formulation of the collection/lysis/transport/storage compositions described herein for the collection of macromolecules such as, proteins, RNA and/or DNA. In an overall sense, the method generally involves combining one or more chaotropes and nuclease-free water at a temperature of about 20°C to 90°C in a reaction zone; then combining the dissolved one or more chaotropes with one or more reducing agents, one or more chelators, and one or more detergents in the reaction zone to form an intermediate composition; optionally combining a silicone polymer with the intermediate composition in an amount sufficient to minimize foaming during further preparation of the one-step aqueous formulation; combining a sufficient amount of buffer to the intermediate composition to maintain a pH of about 6 to 6.9; optionally combining a second chelating agent to the reaction zone; then increasing the temperature of the second intermediate composition to about 60 to 95°C for about 1 to 30 minutes and lowering the temperature to ambient conditions; optionally then combining a C₁₋₆alcohol with the contents of the reaction zone; and optionally adjusting the pH to be about 6.4 to 6.9 and adding the nucleic acid capture material.

The present disclosure also describes a method for preparing one-step aqueous formulations adapted to obtain a population of nucleic acids from a biological sample or specimen. This method generally involves at least the steps of: a) contacting the sample with an amount of the one-step aqueous formulation effective to: i) at least substantially kill or inactivate potentially-infectious pathogens in the sample; ii) at least chemically or physically lyse a portion of cells to release the desired nucleic acids from the sample; and iii) at least substantially inhibit or prevent the released nucleic acids in the sample from further hydrolysis or enzymatic degradation, modification, or inactivation, so as to obtain the population of the desired nucleic acids from the sample.

Preferably, the methods will include at least contacting the sample with an amount of one or more of the disclosed compositions at a temperature of from 0°C to about 40°C (more preferably at a temperature of 4°C to about 35°C, and still more preferably at a temperature of 10°C to about 30°C or ambient temperatures) for a period of time of at least 15 minutes, more preferably at least 30 minutes, and more preferably at least 8 to 24 hours. During contact with the composition and for all times thereafter, the desired nucleic acids remain substantially intact so as to be detectable without incurring substantial deterioration, degradation, enzymatic cleavage, and/or digestion, modification, or other processing.

Preferably, the integrity of a population of nucleic acids released from the sample into the composition are substantially maintained, even when the composition comprising the sample is stored at ambient temperatures, and even for prolonged periods of time, including, without limitation, storage for greater than about 2 days, greater than about 5 days, greater than about 10 days, greater than about 20 days, or even greater than about 30 days or more. Likewise, it is desirable that the integrity of a population of nucleic acids released from the sample into the composition will be substantially maintained, even when the composition comprising the sample is stored at subtropical and tropical temperatures-- even for prolonged periods of time, including, without limitation, storage for greater than or equal to about 5 days, greater than or equal to about 10 days, greater than or equal to about 15 days, or even greater than or equal to about 20, about 25, about 30, about 35, about 40, about 60, or about 90 days or even greater.

In the practice of the present methods, it is preferable that at least one or more biological cells contained within the sample are substantially lysed to release at least a first population or first plurality nucleic acids contained within such cells into the composition. Preferably, the components of the disclosed composition are sufficient to release and thereafter isolate trough affinity binding to a matrix material such a population from all or substantially all of the unwanted cellular/tissue and/or sample debris (including, without limitation, lipids, phospholipids, peptides, proteins, polysaccharides, lipopolysaccharides, polyols, cellular organelles, membrane components, and such like).

It is also desirable in the practice of the present methods that when one or more microbes, viruses, fungi, and/or other pathogens or organisms of interest are present in, on, or about the sample when collected, such microbes, viruses, fungi, and/or other pathogens or organisms of interest will be lysed, sufficiently inactivated, or substantially killed upon contact with the composition, which facilitates safe handling of the sample by the practitioner. Preferably, one or more components of the disclosed composition are effective to render a pathogenic sample substantially or preferably entirely, non-pathogenic without the need for adding additional components to the composition. However, in certain applications, it may also be desirable to include one or more additional anti-microbial, anti-viral, or antifungal agents to the compositions to render them substantially non-pathogenic, and thus, safe for handling by the practitioner.

Preferably, the composition containing the sample is at least sufficiently stable to permit storage of the sample in the composition at ambient, near-ambient, or even colder or warmer conditions at least substantially (or entirely) from the time of specimen or sample collection substantially until the time of analyzing or characterizing at least a first population of macromolecules from within the sample. As used herein, "ambient temperature" can refer to temperatures of about 18°C to 25°C, or in some embodiments, more preferably from about 20°C to about 22°C.

Preferably, composition containing the sample suspected of containing desired nucleic acids will stabilize and isolate the nucleic acids to the extent that they either remain at least substantially non-degraded (*i.e.*, at least substantially stable) even upon prolonged storage of the composition at ambient, refrigerator, or sub-zero temperatures. It will be desirable that this stability provides that at least about 70%, at least about 85%, more preferably at least about 90%, more preferably at least about 95%, or even more preferably, at least about 98% of the nucleic acids contained within the stored sample will not be degraded upon prolonged storage of the sample. In certain embodiments, substantially all of the desired nucleic acids contained within the sample will be stabilized such that their original integrity is preserved during the collection, lysis, storage, and transport of the processed sample.

The present disclosure also describes kits and sample collection systems utilizing the disclosed compositions and collection/storage/transport/isolation solutions described herein. In particular, such sample collection systems may include a collection device, such as a swab, curette, or culture loop; and a collection vessel, such as a vial test tube, or specimen cup, that contains one or more of the compositions disclosed herein. The collection vessel is preferably releasably openable, such that it can be opened to insert the one-step compositions and closed and packaged, opened to insert the sample and optionally a portion of the collection device and closed for storage and transport, or both. The collection vessel may use any suitable releasable or openable mechanism, including without limitation a screw cap, snap top, press-and-turn top, or the like. Such systems may also further optionally include one or more additional reagents, storage devices, transport devices, and/or instructions for obtaining, collecting, lysing, storing, or transporting samples in such systems. In a preferred embodiment, the one-step compositions of the invention may already be disposed in the reaction zone into which the sample may be associated. In such embodiments, the invention requires only a collection device and the collection vessel. The kit preferably includes one or more isolation or extraction elements to help liberate and/or separate one or more populations of nucleic acids contained within the sample from one or more other biomolecules or sample components to obtain at least partially, or substantially purified nucleic acids suitable for identification, detection, or further molecular analysis.

Also, kits may be packaged for commercial distribution, and may further optionally include one or more collection, delivery, transportation, storage and/or isolation devices for sample or specimen collection, handling, or processing. The container(s) for such kits may typically include at least one vial, test tube, flask, bottle, cup, or other suitable container or collection device, into which the composition(s) may be placed, and, preferably, suitably aliquotted for individual specimen collection, transport, and/or storage. The kit may also include a larger container, such as a case, that includes the smaller, individual containers noted above, along with other collection devices, equipment, reagents, instructions, and/or the like. The kit may also optionally include one or more additional buffers, compounds, or compositions, and may also further optionally include one or more instructions detailing use(s) of the kit in either the collection or storage of one or more biological, clinical, diagnostic, environmental, or forensic sample. Optionally, the kit may also further provide instructions for the transport of the sample once placed in one or more of the disclosed compositions, and may even include instructions or additional reagents detailing one or more subsequent analytical methods or assays employing the nucleic acids isolated from the sample or specimen. Such kits may also include multiples of the various collection devices and collection vessels and any other components to be included, so that the kits can be used to collect multiple samples from the same source or different sources. In one commercial application, the kits are packaged in sets of five or more for convenient sale and use.

Optionally, compositions may include an appropriate detectable marker (*i.e.,* a "label") for determining the presence of the macromolecule of interest. A wide variety of appropriate indicator compounds and compositions are known in the art, including, without limitation, fluorescent, radioactive, enzymatic or other ligands, such as avidin/biotin, *etc.,* which are capable of being detected. It may be desirable to employ a fluorescent label or an enzyme tag such as urease, alkaline phosphatase or peroxidase, instead of radioactive or other environmentally less-desirable reagents. In the case of enzyme tags, colorimetric, chromogenic, or fluorigenic indicator substrates are known that can be employed to provide a method for detecting the sample that is visible to the human eye, or by analytical methods such as scintigraphy, fluorimetry, spectrophotometry, and the like, to identify specific macromolecules.

In general, probes described will be useful both as reagents in solution hybridization, as in PCR, for detection of particular nucleic acid sequences, as well as in embodiments employing a solid phase. One well-known amplification method is the polymerase chain reaction (referred to as PCR) which is described in detail in U.S. Patent Nos. 4,683,195, 4,683,202 and 4,800,159. Another method for amplification is the ligase chain reaction ("LCR"), disclosed, *e.g.,* in EPA No. 320 308, and U.S. Patent 4,883,750. Strand Displacement Amplification (SDA) is another method of carrying out isothermal amplification of nucleic acids that involves multiple rounds of strand displacement and synthesis, *i.e.,* nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA. Target specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

### Examples

Once formulated, the stock solutions of PrimeStore™ are stable at 4°C or below for periods of at least one year or more. Formulated PrimeStore™ solutions (PSSs) are stable at ambient temperature (*e.g*., about 20-30°C) for periods of many months. Once a sample is contacted with a PrimeStore™ formulation as disclosed herein, it can reasonably expected to be stored indefinitely at temperatures of 0°C or below, at least one year or more under refrigeration (*e.g.,* ≈ 4°C and at least 30 days or more at ambient temperature *(e.g.,* about 20-30°C), without significant loss of nucleic acid composition, fidelity or integrity of the sample. For example, without limitation, the integrity of a population of polynucleotides obtained from the sample is at least substantially maintained, and preferably entirely maintained without detectable degradation, when the composition comprising the sample is stored at a temperature of from about -20°C to about 40°C, for a period of from about 30 days to about 60 days.

### Example 1 - Formulation of Exemplary Storage Solutions

The present example provides a general formulation of the PSS compositions. Additional formulations of the PSS compositions are exemplified in Examples 2 through 5.

**Formulation Ranges of Exemplary Components for the Preparation of PrimeStore™ Compositions**

| **Compound** | **Final Concentration Range** |
|---|---|
| A chaotrope, *e.g.:* | |
| Guanidine thiocyanate | about 0.5 M to about 6 M |
| *or* Guanidine hydrochloride | about 0.5 M to about 6 M |
| *or* Guanidine isocyanate | about 0.5 M to about 6 M |
| An anionic detergent, *e.g.:* | |
| *N*-lauroyl sarcosine (*inter alia* Na salt) | about 0.15% to about 1% (wt./vol.) |
| *or* Sodium dodecyl sulfate, | about 0.15% to about 1% (wt./vol.) |
| Lithium dodecyl sulfate, | about 0.15% to about 1% (wt./vol.) |
| Sodium glycocholate, | about 0.15% to about 1% (wt./vol.) |
| Sodium deoxycholate, | about 0.15% to about 1% (wt./vol.) |
| Sodium taurodeoxycholate, *or* | about 0.15% to about 1% (wt./vol.) |
| Sodium cholate | about 0.1% to about 1% (wt./vol.) |
| A reducing agent, *e.g.:* | |
| TCEP | about 0.5 mM to about 30 mM |
| *or* β-ME, DTT, formamide, *or* DMSO | about 0.05 M to about 0.3 M |
| 4. A chelator, *e.g.:* | |
| Sodium citrate | about 0.5 mM to about 50 mM |
| *or* EGTA, HEDTA, DTPA, NTA, APCA, *etc.* | about 0.01 mM to about 1 mM |
| A buffer (*e.g.,* TRIS, HEPES, Bis-Tris, *etc*.) | about 1 mM to about 1 M |
| An acid (*e.g.,* HCl or citric acid) | *q.s.* to adjust to a pH of about 6 to 7, preferably about 6.4 to 6.8 |
| Nuclease-free water | *q.s.* to desired final volume |
| Optionally one or more of: | |
| A surfactant/defoaming agent, *e.g.:* | |
| Antifoam A® or Tween® | about 0.0001% to about 0.3% (wt./vol.) |
| An alkanol (*e.g.,* methanol, ethanol, propanol, *etc*.) | about 1% to about 25% (vol./vol.) |
| A carrier/IPC RNA or DNA | about 1 pg to about 1 µg/mL |
| Magnetic NACM | about 1 ng to 10 µg per 100 µL |

Guanidine thiocyanate, sodium citrate, Antifoam A® Concentrate, and *N*-lauroylsarcosine, sodium salt, were all purchased from Sigma Chemical Co. (St. Louis, MO, USA). Tris(2-carboxyethyl) phosphine hydrochloride (TCEP) was obtained from Soltec Ventures Inc. (Beverly, MA, USA). 2-amino-2-hydroxymethyl-propane-1,3-diol (TRIS) was obtained from Applied Biosystems/Ambion (Austin, TX, USA). 2-[2-(Bis(carboxymethyl)amino)ethyl-(carboxymethyl)amino]acetic acid (EDTA) GIBCO® Ultra Pure was obtained from Invitrogen Corp. (Carlsbad, CA, USA). All other reagents are available commercially from Sigma-Aldrich or USB Corporation.

### Example 2 - Formulation of an Exemplary Storage Solution

The present example describes a first exemplary formulation of the compositions of the invention. This formulation is alternatively referred to as "PrimeStore™ Solution" or "PSS" *version 1.*

**Preparation of PrimeStore™ Composition (Version 1)**

| **Compound** | **Final Conc.** |
|---|---|
| Guanidine thiocyanate | 4 M |
| Sodium citrate | 30 mM |
| Sodium dodecyl sulfate | 0.25% (wt./vol.) |
| *N*-lauroyl sarcosine, sodium salt | 0.25% (wt./vol.) |
| 2-mercaptoethanol (β-ME) | 0.1 M |
| Antifoam A | 0.1% (wt./vol.) |
| Citric acid | *q.s.* to adjust pH to 6.5 |
| Nuclease-free water | 11.82 mL |
| Magnetic NACM beads | 1 µg |

### Example 3 - Preparation of A Second Exemplary Storage Solution

The present example describes the preparation of another storage solution. This formulation may be referred to as PSS or PrimeStore™ *version 2.*

**Preparation of PrimeStore™ Composition (Version2)**

| **Compound** | **Quantity** | **Final Conc.** |
|---|---|---|
| Guanidine thiocyanate | 35.488 gm | 3 M |
| TCEP | 0.02867 gm | 1 mM |
| Sodium citrate | 0.2931 gm | 10 mM |
| *N*-lauroyl sarcosine, sodium salt (NLS) | 0.5 gm | 0.5% |
| Antifoam A (10% solution) | 200 µL | 0.002% |
| TRIS (1 M) | 10 mL | 100 mM |
| EDTA (0.5 M) | 20 µL | 0.1 mM |
| Hydrochloric acid (HCl) | *q.s.* to adjust pH to 6.7 | -- |
| Nuclease-free water | *q.s.* to 100 mL | -- |
| Magnetic NACM beads | 1 µg | -- |

### Example 4 - Preparation of A Third Exemplary Storage Solution

The present example describes the preparation of another storage solution. This formulation may be referred to as PSS or PrimeStore™ *version 2.2.*

**Preparation of PrimeStore™ Composition (Version 2.2)**

| **Compound** | **Quantity** | **Final Conc.** |
|---|---|---|
| Guanidine thiocyanate | 35.488 gm | 2.5 M |
| TCEP | 0.02867 gm | 0.5 mM |
| Sodium citrate | 0.2931 gm | 10 mM |
| *N*-lauroyl sarcosine, sodium salt (NLS) | 0.5 gm | 0.4% |
| Antifoam A (10% solution) | 200 µL | 0.002% |
| TRIS (1 M) | 10 mL | 100 mM |
| EDTA (0.5 M) | 20 µL | 0.1 mM |
| Ethanol, molecular grade (96-100%) | 23 mL | 23% (vol./vol.) |
| Hydrochloric acid (HCl) | *q.s.* to adjust pH to 6.7 | -- |
| Nuclease-free water | *q.s.* to 100 mL | -- |
| Magnetic NACM beads | 1 µg | -- |

### Example 5

The inactivation of gram positive mycobacteria from sputum or other mucous-containing specimens that are contaminated with other organisms is difficult since *mycobacteria* are often shielded by the highly viscous sputum mucous content within the sample. The components present in PSS safely inactivate 10⁸ concentrations of mycobacterium through chemical inactivation/killing by phospholipid membrane shearing, protein denaturation, chelation and buffering effect. However, mycobacteria present in collected primary sputum samples that contain human serum albumin, mucopolysaccharide and mucoprotein fractions may shield and protect the organism from direct inactivation by chemical inactivation mechanisms in PSS. Additional strategies to enhance mycobacteria inactivation from primary sputum samples collected in PSS include physical agitation using various sized porcelain, ceramic, plastic or glass beads. When combined with vortexing, the physical disruption of sputum matrix exposes the organism to PSS and facilitate greater inactivation. Furthermore, the same or different beads that are coated with silica dioxide and magnetized are used to bind and concentrate released DNA and RNA from inactivated cells and microbes though the chemical processes of PSS. The prerequisites for high affinity attachment of nucleic acid (DNA/RNA) to silica are: 1) a preferred pH of 6.8-7.0, and 2) a preferred presence of a chaotrophic agent. Both of these preferences are existing features of PSS formulation. PSS containing beads facilitates killing of mycobacteria in primary sputum samples. The further addition of magnetized, silica coated beads used in collection tubes and in combination with a magnetic stand concentrates nucleic acids from primary samples as well. In addition to physical agitation through bead-based vortex, the addition of heating (within a temperature range of 50-90C) and or sonication enhances inactivation and killing of mycobacteria in primary sputum samples.

### Example 6 Exemplary Protocol for the Preparation of PSS

40 mL of nuclease-free water is first added to a clean beaker with a stir bar. The beaker is placed on a hot plate/stirrer and adjusted temperature to 60 - 65°C. Stirring speed is set to medium and 35.488 gm of guanidine thiocyanate is added slowly to the water allowing it to dissolve as added. Next, 0.0287 gm of TCEP is added to beaker and stirrer speed increased to help dissolve crystals. 0.2931 gm of sodium citrate is added to the beaker followed by 0.5 gm of NLS to the solution. Stirrer speed is then increased again to create a vortex in the beaker. This vortex pulls the NLS into the solution and helps to dissolve reagent. A prepared 10% Antifoam A solution (1 mL Antifoam A Concentrate + 9 mL nuclease-free water) is vortexed and 200 µL of the 10% Antifoam A is pipette into the solution. Next, 10 mL of 1 M TRIS is added into the solution and 20 µL of 0.5 M EDTA. Temperature is increased to bring the solution to 75-80°C with stirring for 3-5 minutes. The beaker is removed from heat and the solution allowed to cool to room temperature (≈22-25°C). 23 mL of ethanol is added to the solution and mixed thoroughly, and the pH is adjusted to 6.9 with HCl. Solution is poured into a clean 100 mL graduated cylinder. 1 µg magnetic affinity matrix beads is added along with nuclease-free water to bring total volume to 100 mL. The solution is transferred to a labeled sterile container and stored at room temperature (≈22-25°C). Preferably each reagent is completely dissolved before adding the next.

### Example 7 Protocol for the Preparation of PrimeStore with Magnetic Beads Extraction

PrimeStore MTM was prepared as a 1.5mL solution in 5mL cryovial tubes. Two types of commercially available silica coated, magnetized beads (MagAttract Material No. 1031699, Qiagen, and Agencourt, Beckman Coulter, Inc) were used for this example. A total of 100 µL of beads was added to PrimeStore MTM tubes. A purified strain of *Mycobacterium tuberculosis* (H27Rv) that was inactivated by gluteraldehyde and sonication was used at a concentration of 10³ CFU/mL. *Mycobacterium tuberculosis* (MTB) was added to PrimeStore tubes containing magnetic beads and PrimeStore Tubes without magnetic beads (control). PrimeStore tubes were vortexed 5-10 seconds and tubes with beads were subsequently attached on top of a magnet for 2 minutes to attract tubes with beads to the bottom. While on the magnet, the PrimeStore tubes were uncapped and the solution was removed. Tubes were removed from the magnet and 200µL of nuclease free water was added to the beads. Each tube was vortexed to elute nucleic acids from the beads. The PrimeStore tube was placed back on the magnet for 2 minutes to draw beads to the bottom. The eluate was transferred into a microcentrifuge tube. 200 µL of PrimeStore (without magnetic beads) pluscontaining MTB was removed and placed into a microcentrifuge tube to serve as an unconcentrated control. The 200 ul aliquots from bead-concentrated and control tubes were subjected to nucleic acid extraction using the Qiagen DNA Mini Kit (Qiagen Inc., Valencia, CA). Purified nucleic acids from both extraction procedures were amplified in replicate fashion using a real-time PCR assay on the ABI 7500 Real Time PCR System. Detection from PrimeStore MTM with magnetic beads had replicate C_{T} values 28.50, whereas detection from PrimeStore MTM without magnetic beads had replicate C_{T} values of 29.78. The decrease in C_{T} values from samples that were concentrated using glass beads indicates a higher initial template starting concentration. These results demonstrate that silica coated beads and likely other affinity matrix materials as an example for any silica coated matrix are compatible with PrimeStore MTM solution. Also, capture with magnetic beads allows an increase in concentration of nucleic acids from the sample prior to extraction, and enhances detection sensitivity. This allows for the concentration of nucleic acids from low-level samples for the potential detection of the presence or absence of pathogens in biological samples. Thus, the methodology is a simplified and efficient approach for concentrating minute quantities of nucleic acids (RNA and DNA) from collected specimens prior to extraction.

## Claims

1. An aqueous composition for use in extracting *Mycobacterium tuberculosis* nucleic acids from biological samples, comprising:
one or more chaotropes;
one or more detergents;
one or more reducing agents;
one or more chelators;
one or more buffers;
a defoaming agent, and
one or more nucleic acid capture matrix (NACM) materials,
together present in an amount sufficient to denature proteins, inactivate enzymes, kill pathogens, and not degrade nucleic acids of a sample suspected of containing selected nucleic acid sequences when a sample is contacted with the composition; wherein: a) the one or more chaotropes comprise 4 M guanidine thiocyanate; b) the one or more detergents comprise 0.25% (wt./vol.) sodium dodecyl sulfate and 0.25% (wt./vol.) *N*-lauroyl sarcosine, sodium salt; c) the one or more reducing agents comprise 0.1 M 2-mercaptoethanol; d) the one or more chelators comprise 30 mM sodium citrate; e) the one or more buffers are present in an amount from 0.0001% to 0.3% (wt./vol.) or from 1 mM to 1M; f) the defoaming agent comprises 0.1% silicone polymer (wt./vol.); and (g) the one or more NACM materials which comprise polymer, porcelain, ceramic, plastic, polymer or glass beads coated with a nucleic acid binding substance, or a combination thereof, and are present in an amount from 1 ng to 10 µg per 100 µL.

2. A composition as claimed in claim 1, further comprising the sample suspected of containing microbes or cells.

3. A composition as claimed in claim 1 or claim 2, further comprising (a) one or more short-chain alkanols; preferably that comprise methanol, ethanol, propanol, butanol, pentanol, hexanol, or any combination thereof and which are present in an amount from 1 to 25% (vol./vol.).

4. A composition as claimed in any of claims 1 to 3, buffered to a pH of 6 to 7.

5. A composition as claimed in any preceding claim, further comprising predetermined nucleic acid sequence(s) as internal positive and/or negative control.

6. A composition as claimed in any of claims 2 to 5, comprising one or more lysed viruses and/or bacterial, fungal, animal, or plant cells.

7. A method of detecting the presence or absence of a nucleotide sequence, gene, or genome in a biological sample suspected of containing *Mycobacterium tuberculosis,* comprising:
in one step contacting the biological sample with an amount of an aqueous composition in accordance with any of claims 1 to 6 that inactivates enzymes, kills the cells microbes and physically lyses the cells of the biological sample releasing nucleic acids from the cells;
collecting the nucleic acid capture matrix material thereby in one step isolating and concentrating the released nucleic acid;
performing a nucleic acid test on the isolated and concentrated nucleic acids; and
determining the presence or absence of a specific nucleic acid sequence, gene or genome that is specific to *Mycobacterium tuberculosis,* from the nucleic acid testing to determine the presence or absence of the specific nucleotide sequence in the biological sample.

8. A method as claimed in claim 7, wherein:
(a) the integrity of nucleic acids is at least substantially maintained when the aqueous composition comprising the sample is stored at a temperature of from 15 °C to 40 °C for a period of from 7 to 14 days; or
(b) the aqueous composition comprising the sample is stored substantially at ambient temperature from the time of collection to the time of determining the presence or absence of the nucleic acid sequence.

## Patentansprüche

1. Wässrige Zusammensetzung zur Verwendung beim Extrahieren von *Mycobacterium tuberculosis*-Nukleinsäuren aus biologischen Proben, wobei die Zusammensetzung folgendes umfasst:
ein oder mehrere chaotrope Substanzen;
eine oder mehrere Detergenzien;
ein oder mehrere Reduktionsmittel;
einen oder mehrere Chelatoren;
einen oder mehrere Puffer;
ein Entschäumungsmittel; und
eine oder mehrere Nucleic Acid Capture Matrix (NACM)-Substanzen;
die gemeinsam in einer ausreichenden Menge vorhanden sind, um Proteine zu denaturieren, Enzyme zu inaktivieren, Pathogene zu töten und Nucleinsäuren einer Probe nicht zu verschlechtern, für die angenommen wird, dass sie ausgewählte Nukleinsäuresequenzen enthält, wenn eine Probe mit der Zusammensetzung in Kontakt gebracht wird, wobei: a) die eine oder die mehreren chaotropen Substanzen 4 M Guanidinthiocyanat umfassen; b) die eine oder mehreren Detergenzien 0,25 % (Gew./Vol.) Natriumdodecylsulfat und 0,25 % (Gew./Vol.) *N*-Lauroylsarcosin, Natriumsalz umfassen; c) das eine oder die mehreren Reduktionsmittel 0,1 M 2-Mercaptoethanol umfassen; d) der eine oder die mehreren Chelatoren 30 mM Natriumcitrat umfassen; e) der eine oder die mehreren Puffer in einer Menge zwischen 0,0001 % bis 0,3 % (Gew./Vol.) oder zwischen 1 mM und 1 M vorhanden sind; das Entschäumungsmittel 0,1 % Silikonpolymer (Gew./Vol.) umfasst; und (g) die eine oder mehreren NACM-Substanzen, die Polymer, Porzellan, Keramik, Kunststoff, Polymer oder Glaskügelchen umfassen, die mit einer Nukleinsäurebindungssequenz überzogen sind, oder eine Kombination davon, und die in einer Menge von 1 ng bis 10 µg pro 100 µL vorhanden sind.

2. Zusammensetzung nach Anspruch 1, die ferner die Probe umfasst, für die angenommen wird, dass sie Mikroben oder Zellen enthält.

3. Zusammensetzung nach Anspruch 1 oder 2, die ferner folgendes umfasst: (a) ein oder mehrere kurzkettige Alkanole; die vorzugsweise Methanol, Ethanol, Propanol, Butanol, Pentanol, Hexanol oder eine beliebige Kombination davon umfassen und die in einer Menge von 1 bis 25 % (Vol./Vol.) vorhanden sind.

4. Zusammensetzung nach einem der Ansprüche 1 bis 3, auf einen pH-Wert von 6 bis 7 gepuffert.

5. Zusammensetzung nach einem der vorstehenden Ansprüche, wobei diese ferner eine/mehrere vorbestimmte Nukleinsäuresequenz(en) als interne positive und/oder negative Kontrolle umfasst.

6. Zusammensetzung nach einem der Ansprüche 2 bis 5, wobei diese ferner einen oder mehrere lysierte Viren und/oder bakterielle, Pilz-, tierische oder pflanzliche Zellen umfassen.

7. Verfahren zum Nachweisen des Vorhandenseins oder Fehlens einer Nukleotidsequenz, eines Gens oder eines Genoms in einer biologischen Probe, für die angenommen wird, dass sie *Mycobacterium tuberculosis* enthält, wobei das Verfahren folgendes umfasst:
in einem Schritt, Inkontaktbringen der biologischen Probe mit einer Menge einer wässrigen Zusammensetzung gemäß einem der Ansprüche 1 bis 6, welche Enzyme inaktiviert, die Zellmikroben tötet und physisch die Zellen der biologischen Probe lysiert, wobei Nukleinsäuren von den Zellen freigesetzt werden;
Sammeln der Nucleic Acid Capture Matrix-Substanz, wodurch in einem Schritt die freigesetzte Nukleinsäure isoliert und konzentriert wird;
Durchführen eines Nukleinsäuretests an den isolierten und konzentrierten Nukleinsäuren; und
Bestimmen des Vorhandenseins oder Fehlens einer spezifischen Nukleinsäuresequenz, eines Gens oder eines Genoms, das spezifisch ist für *Mycobacterium tuberculosis,* aus den Nukleinsäuretests, um das Vorhandensein oder Fehlen der spezifischen Nukleotidsequenz in der biologischen Probe zu bestimmen.

8. Verfahren nach Anspruch 7, wobei:
(a) die Integrität der Nukleinsäuren im Wesentlichen aufrechterhalten wird, wenn die wässrige Zusammensetzung, welche die Probe umfasst, über einen Zeitraum von 7 bis 14 Tagen auf einer Temperatur von 15 °C bis 40 °C gelagert wird; oder
(b) die wässrige Zusammensetzung, welche die Probe umfasst, im Wesentlichen bei Raumtemperatur gelagert wird ab dem Zeitpunkt des Sammelns bis zum Zeitpunkt des Bestimmens des Vorhandenseins oder des Fehlens der Nukleinsäuresequenz.

## Revendications

1. Composition aqueuse destinée à être utilisée pour extraire les acides nucléiques de *Mycobacterium tuberculosis* à partir d'échantillons biologiques, comprenant :
au moins un chaotrope ;
au moins un détergent ;
au moins un agent réducteur ;
au moins un agent chélateur ;
au moins un tampon ;
un agent antimousse, et
au moins un matériau de la matrice de capture des acides nucléiques (NACM),
ensemble présents en une quantité suffisante pour dénaturer les protéines, inactiver les enzymes, tuer les agents pathogènes et ne pas dégrader les acides nucléiques d'un échantillon supposé contenir des séquences d'acides nucléiques sélectionnées lorsqu'un échantillon est en contact avec la composition ; a) l'au moins un chaotrope comprenant 4 M de thiocyanate de guanidine ; b) l'au moins un détergent comprenant 0,25 % (p/vol) de dodécyle de sodium et 0,25 % (p/vol) de *N*-lauroyl sarcosine, sel de sodium ; c) l'au moins un agent réducteur comprenant 0,1 M de 2-mercaptoéthanol ; d) l'au moins un chélateur comprenant 30 mM de citrate de sodium ; e) l'au moins un tampon étant présent en une quantité comprise entre 0,0001 % et 0,3 % (poids/vol) ou entre 1 mM et 1M ; f) le produit antimousse comprenant 0.1 % de polymère de silicone (poids/vol.) ; et (g) l'au moins un matériau NACM comprenant des billes de polymère, de porcelaine, de céramique, de plastique, de polymère ou de verre revêtues d'une substance liant un acide nucléique, ou une combinaison de celles-ci, et étant présents en une quantité de 1 ng à 10 µg pour 100 µL.

2. Composition selon la revendication 1, comprenant en outre l'échantillon supposé contenir des microbes ou des cellules.

3. Composition selon la revendication 1 ou 2, comprenant en outre (a) au moins un alcanol à chaîne courte ; qui comprennent de préférence du méthanol, de l'éthanol, du propanol, du butanol, du pentanol, de l'hexanol ou toute combinaison de ceux-ci et sont présents en une quantité de 1 à 25 % (volume/vol).

4. Composition selon l'une quelconque des revendications 1 à 3, mise en tampon à un pH de 6 à 7.

5. Composition selon l'une quelconque des revendications précédentes, comprenant en outre une ou plusieurs séquences d'acide nucléique prédéfinies comme témoin positif et/ou négatif interne.

6. Composition selon l'une quelconque des revendications 2 à 5, comprenant au moins un virus lysés et/ou des cellules bactériennes, fongiques, animales ou végétales.

7. Procédé de détection de la présence ou de l'absence d'une séquence nucléotidique, d'un gène ou d'un génome dans un échantillon biologique supposé contenir *Mycobacterium tuberculosis,* comprenant les étapes consistant à :
en une étape, mettre en contact l'échantillon biologique avec une quantité d'une composition aqueuse selon l'une quelconque des revendications 1 à 6 qui inactive les enzymes, tue les microbes des cellules et lyse physiquement les cellules de l'échantillon biologique libérant les acides nucléiques des cellules ;
collecter le matériau de la matrice de capture de l'acide nucléique, ce qui permet, en une étape, d'isoler et de concentrer l'acide nucléique libéré ;
effectuer un test d'acide nucléique sur les acides nucléiques isolés et concentrés ; et
déterminer la présence ou l'absence d'une séquence, d'un gène ou d'un génome d'acide nucléique spécifique de *Mycobacterium tuberculosis,* à partir de l'analyse des acides nucléiques pour déterminer la présence ou l'absence de la séquence nucléotidique spécifique dans l'échantillon biologique.

8. Procédé selon la revendication 7,
(a) l'intégrité des acides nucléiques étant au moins sensiblement maintenue lorsque la composition aqueuse constituant l'échantillon est conservée à une température comprise entre 15 °C et 40 °C pendant une période de 7 à 14 jours ; ou
(b) la composition aqueuse comprenant l'échantillon étant stockée sensiblement à la température ambiante à partir du moment de la collecte jusqu'au moment de la détermination de la présence ou de l'absence de la séquence d'acide nucléique.
